# EUROPEAN PATENT SPECIFICATION

(11) **EP 2 966 449 B1**
(45) Date of publication and mention of the grant of the patent: **29.04.2020**
(21) Application number: 14760124.9
(22) Date of filing: 05.03.2014
(51) Int. Cl.: G01N 33/68

(54) **KIT FOR DIAGNOSING KIDNEY DISEASES**
KIT ZUR DIAGNOSE VON NIERENERKRANKUNGEN
ENSEMBLE POUR DIAGNOSTIQUER DES MALADIES RÉNALES

(30) Priority: 05.03.2013 KR 20130023611; 02.07.2013 KR 20130077034
(43) Date of publication of application: 13.01.2016
(73) Proprietor: Kyungpook National University Industry-Academic Cooperation Foundation, Daegu 702-701 (KR)
(72) Inventor: PARK, Kwon Moo, Daegu 706-935 (KR); KIM, Jee In, Daegu 701-749 (KR); PARK, Sun Hee, Daegu 706-776 (KR); JANG, Hee Seong, Gumi-si Gyeongsangbuk-do 730-833 (KR)
(74) Representative: ABG Intellectual Property Law, S.L.
(86) International application number: PCT/KR2014/001813
(87) International publication number: WO 2014/137158

(56) References cited:
- WO-A1-03/048774
- WO-A2-2004/018702
- WO-A2-2007/012628
- US-A1- 2009 105 173
- US-A1- 2012 010 230
- R. RADFORD ET AL: "Carcinogens induce loss of the primary cilium in human renal proximal tubular epithelial cells independently of effects on the cell cycle", AMERICAN JOURNAL OF PHYSIOLOGY: RENAL PHYSIOLOGY, vol. 302, no. 8, 18 January 2012 (2012-01-18), pages F905-F916, XP055292731, United States ISSN: 1931-857X, DOI: 10.1152/ajprenal.00427.2011
- SANDER G BASTEN ET AL: "Reduced cilia frequencies in human renal cell carcinomas versus neighboring parenchymal tissue", CILIA, BIOMED CENTRAL LTD, LONDON, UK, vol. 2, no. 1, 31 January 2013 (2013-01-31), page 2, XP021140459, ISSN: 2046-2530, DOI: 10.1186/2046-2530-2-2
- M. C. HOGAN ET AL: "Characterization of PKD Protein-Positive Exosome-Like Vesicles", JOURNAL OF THE AMERICAN SOCIETY OF NEPHROLOGY., vol. 20, no. 2, 28 January 2009 (2009-01-28), pages 278-288, XP055225650, US ISSN: 1046-6673, DOI: 10.1681/ASN.2008060564
- HOORN ET AL: "Prospects for urinary proteomics: exosomes as a source of uri nary biomarkers", NEPHROLOGY, BLACKWELL SCIENCE, CARLTON, AU, vol. 10, no. 3, 1 June 2005 (2005-06-01), pages 283-290, XP008139623, ISSN: 1320-5358, DOI: 10.1111/J.1440-1797.2005.00387.X [retrieved on 2005-04-01]
- HIROAKI ISHIKAWA ET AL: "Proteomic Analysis of Mammalian Primary Cilia", CURRENT BIOLOGY, CURRENT SCIENCE, GB, vol. 22, no. 5, 16 January 2012 (2012-01-16), pages 414-419, XP028465443, ISSN: 0960-9822, DOI: 10.1016/J.CUB.2012.01.031 [retrieved on 2012-01-18]
- VERGHESE, E. ET AL.: 'Renal cilia display length alterations following tubular injury and are present early in epithelial repair.' NEPHROL DIAL TRANSPLANT vol. 23, 2008, pages 834 - 841, XP055284068
- DEANE. J-A. ET AL.: 'Visualizing renal primary cilia.' NEPHROLOGY vol. 18, 2013, pages 161 - 168, XP028967359
- WANG, S. ET AL.: 'The autosomal recessive polycystic kidney disease protein is localized to primary cilia, with concentration in the basal body area.' J AM SOC NEPHROL. vol. 15, 2014, pages 592 - 602, XP055284070

## Description

### [Technical Field]

The present invention relates to methods for diagnosing the occurrence or progress of a renal disease. Also disclosed are a composition for urinalysis for diagnosing a renal disease; a kit for urinalysis for diagnosing a renal disease; and a method for detecting primary cilia or a component thereof isolated from renal tissues, or basal bodies of the primary cilia or a component thereof isolated from renal tissues.

### [Background Art]

Kidneys are excretory organs in vertebrates that serve as a filter to excrete waste in the blood in the form of urine. Kidneys largely consist of three parts, i.e., the outer cortex, the medulla located inside, and the renal pelvis inside the medulla. Among them, the medulla consists of renal tubules and collecting ducts formed by the tubules, and each end of the collecting ducts are open at the renal pelvis. In particular, the renal tubules consist of mutually different types of cells depending on the sections; i.e., from the nearest region to the glomerulus, they are called proximal tubules, thin descending tubules, thin ascending tubules, distal tubules, and collecting ducts in this order. Urine, which is produced while a glomerulus filtrate filtered in the glomerulus is absorbed or released as it passes through these renal tubules, is stored in the urinary bladder and then excreted through the urethra. These renal tubules may show mutually different sensitivities to renal injuries such as an ischemia/reperfusion (I/R) injury. Since kidneys can serve their roles without cessation, a temporary block of their roles or occurrence of a disease thereon may cause a fatal result. Accordingly, there has been a need for the development of a technology for a rapid and simple diagnosis of renal diseases.

Meanwhile, primary cilia are microtubule-based non-motile organelles that extend from the surface of mammalian cells. In the kidneys, primary cilia are found on the cell surface of all renal tubular epithelial cells, glomeruli, and lumen of glomerular capsule cells, except for the intercalated cells of the collecting tubules. Primary cilia act as chemo- and mechanosensory organelles essential for responding to fluid flow and chemical factors and maintaining the structure and functions of the nephron. Primary cilia are known to be associated with proliferation and differentiation of renal epithelial cells under various pathophysiological conditions including polycystic kidney disease. However, it has not been known whether they can be used as an index for diagnosing renal diseases from urine.

US 2012/010230 A1 discloses the use of primary cilia, detectable by anti-α-tubulin antibodies, for the stratification of cancer, based on the finding that some cancer forms are associated with the expression of primary cilia on cancerous cells. Basten, S. G. et al. (Cilia, 2013, Vol. 2, No. 1, page 2) teach the immunohistochemical analysis of renal cancer tissue using anti-α-tubulin antibodies and report a reduced frequency of primary cilia in tumor tissue versus normal renal tissue. Verghese, E. et al. (Nephrol. Dial. Transplant., 2008, Vol. 23, pages 834-841) investigate the influence of ischemia on renal tissue by way of immunohistochemistry and report that tubular injury is associated with changes in cilial length.

### [Disclosure]

### [Technical Problem]

The present inventors, while endeavoring to develop a method for a rapid and simple diagnosis of renal diseases with kidney-specificity and sensitivity, first discovered that primary cilia, proteins constituting the primary cilia, or basal bodies of the primary cilia (primary cilia support) were excreted through the urine of patients with various renal diseases by detecting the components constituting the primary cilia and the fragments thereof, and also confirmed that the primary cilia or basal bodies of the primary cilia were not discovered in a normal control group. Specifically, it was confirmed that the presence of a renal disease can be diagnosed with a small amount of urine of about 10 µL. More specifically, the detection of proteins constituting primary cilia, including fragments of primary cilia isolated from the kidney, and factors constituting these primary cilia, including acetylated tubulin, only refers to the case when they were detached from the renal tubular cells, and it was confirmed that the detection can have high sensitivity and specificity in diagnosing renal diseases, thereby completing the present invention.

### [Technical Solution]

An object of the present disclosure is to provide a composition for urinalysis for diagnosing a renal disease, containing an agent for specifically detecting primary cilia or a component thereof isolated from renal tissues, or basal bodies of the primary cilia or a component thereof isolated from renal tissues.

Another object of the present disclosure is to provide a kit for urinalysis for diagnosing a renal disease, including a substrate for adsorbing to or receiving primary cilia or a component thereof isolated from renal tissues, or basal bodies of the primary cilia or a component thereof isolated from renal tissues; or isolating primary cilia or basal bodies of the primary cilia isolated from renal tissues, from urine.

A further object of the present disclosure is to provide a method for detecting primary cilia or a component thereof isolated from renal tissues, or basal bodies of the primary cilia or a component thereof isolated from renal tissues, including confirming the presence of the primary cilia or a component thereof isolated from renal tissues, or the basal bodies of the primary cilia or a component thereof isolated from renal tissues, from an isolated urine sample.

Finally, the object of the present invention is to provide a method for diagnosing the occurrence or progress of a renal disease, including confirming the presence of primary cilia or a component thereof isolated from renal tissues, or basal bodies of the primary cilia or a component thereof isolated from renal tissues; the concentrations thereof; or the length of the primary cilia thereof, from an isolated urine sample. Thus, the invention relates to a method for diagnosing the occurrence or progress of a renal disease using a urine sample, comprising;
(i) contacting an isolated urine sample with (a) a binding agent specific for acetylated α-tubulin and, optionally, with (b) a binding agent specific for ARL13B, and
(ii) detecting the acetylated α-tubulin and, optionally, ARL13B; or the concentrations thereof from the isolated urine sample.

### [ADVANTAGEOUS EFFECTS OF THE INVENTION]

The method for urinalysis of the present invention have the advantages of rapidly and simply identifying the presence of a renal disease by detecting the presence of primary cilia or a component thereof or proteins constituting the same from the urine of a renal disease patient.

### [BRIEF DESCRIPTION OF THE DRAWINGS]

FIG. 1 shows a diagram illustrating the morphology of primary cilia and the proteins of primary cilia.
FIG. 2 shows the results of immunofluorescence staining of a urine sample, obtained 24 hours after ischemia/reperfusion (I/R) injury, with Arl13b antibodies, a constituting component of the basal bodies of the primary cilia (left - control; right - ischemia/reperfusion injury group).

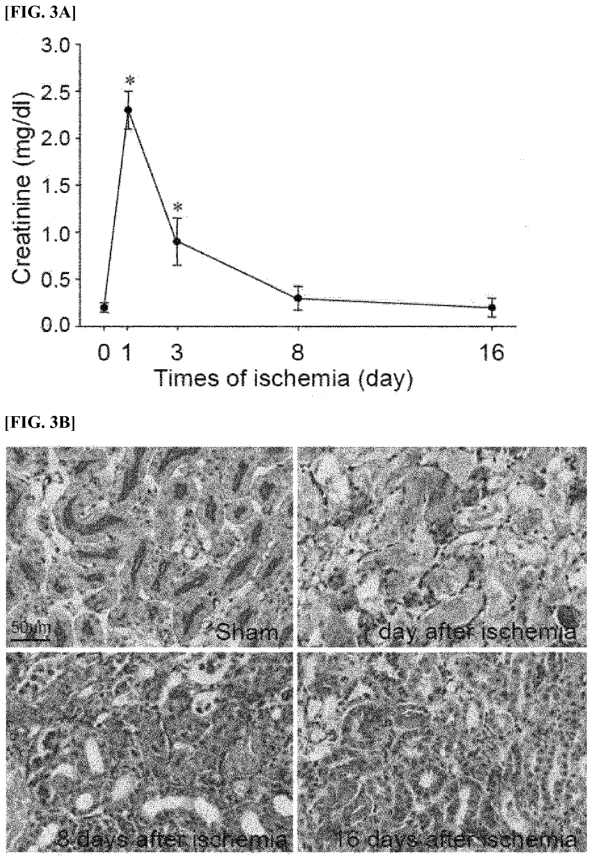

FIGS. 3A and 3B show the results of measurement of renal morphology and measurement of concentrations of plasma creatinine after ischemia/reperfusion injury, respectively. On the 0^{th} day, mice were subjected to a bilateral renal ischemia or sham operation for 30 minutes. (A) The concentration in plasma creatinine of the mice according to time was confirmed (n=6 per time point). The results are expressed as mean ± SE. * P < 0.001 vs. respective 0 day (before surgery). (B) PAS-stained kidney sections are shown. The kidney sections corresponding to a sham operation, 1 day after ischemia, 8 days after ischemia, and 16 days after ischemia are shown.
FIGS. 4A to 4E show images and graphs illustrating the changes in primary cilia after ischemia/reperfusion injury.

Specifically, mice were subjected to bilateral renal ischemia for 30 minutes, and the kidneys were harvested prior to ischemia, 1 day after ischemia, 8 days after ischemia, and 16 days after ischemia, respectively.
(A) The kidney sections were double-stained with anti-acetylated α-tubulin antibodies and other antibodies (anti-Na+-K+-ATPase antibodies (b, d), anti-AQP-1 antibodies (c), or anti-AQP-2 antibodies (e)). In particular, PI indicates stained nuclei (blue). For length measurement, lengths of 30 cilia per kidney were measured (n=3) and averaged. Results are expressed as mean ± SE. The arrows in each image indicate primary cilia.
(B) Images photographed from the cortex and the outer medulla are shown. Na/K-ATPase (green) was more strongly expressed in the distal tubules than in the proximal tubules. Specifically, 1 day after ischemia, the cilia on the tubule cells in the outer medulla were shown to be more severely disrupted than those in the cortex. Primary cilia (green) were observed in the lumen, the length of cilia varied depending on the regions (renal tubules). The changes in cilia length were more dramatic in the outer medulla than in the cortex.
(C) Images photographed from the outer medulla and the inner medulla are shown, respectively. The length of the cilia of AQP-1-positive (red) descending thin limb of Henle cells, after ischemia, did not change according to the location in the inner or outer medulla. The cilia length was normal 1 day after the ischemia, increased 8 days after the ischemia, and returned to normal 16 days after the ischemia.
(D) Images of distal tubule cells photographed from the cortex and the outer medulla are shown. Na/K-ATPase (green) was strongly expressed in the distal tubule cells. Primary cilia (green) were observed in the lumen. The cilia length was normal one day after the ischemia, increased 8 days after the ischemia, and returned to normal 16 days after the ischemia.
(E) Images photographed for the inner and outer medulla and the cortex are shown. AQP2 (red) was used as a marker for collecting duct cells. The length of primary cilia in the collecting duct cells changed depending on their locations and post-ischemic time. The cilia length in the collecting duct cells decreased in the cortex and the outer medulla 1 day after ischemia, increased in the cortex, the outer medulla, and the inner medulla 8 days after ischemia, and was normal 16 days after ischemia. * p < 0.05 vs. day 0; #, p < 0.05 vs. other groups at day 1; $, p < 0.05 vs. other groups at day 8 or 16.

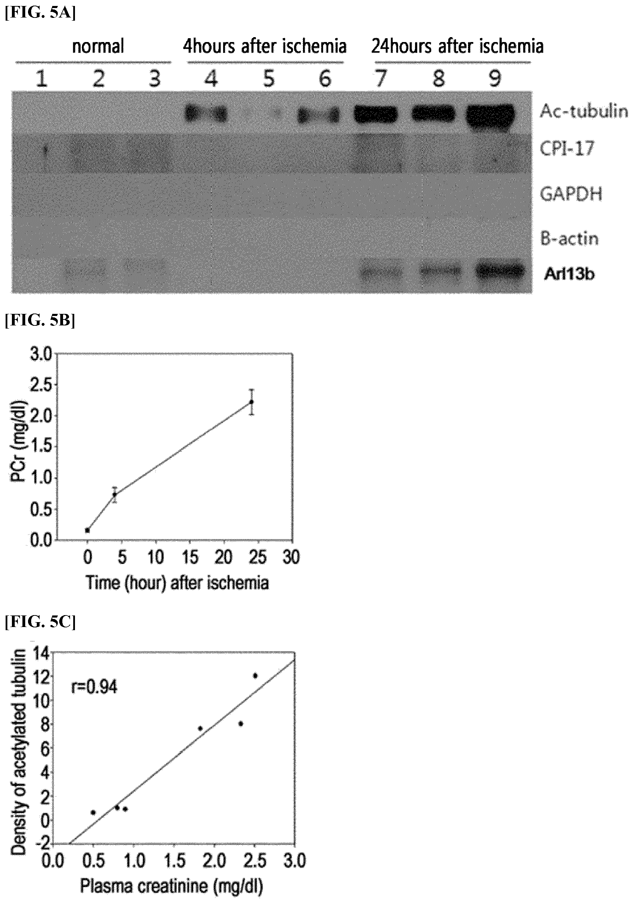

FIG. 5A shows the result of Western blot analysis of 10 µL of a urine sample collected at the indicated time, performed using anti-acetylated α-tubulin antibodies, anti-arl13b antibodies, anti-GAPDH antibodies, anti-CPI-17 antibodies, and anti-β-actin antibodies (n=4). The expression of acetylated α-tubulin and arl13b increased with the increase in renal injury. However, other proteins which constitute the cytoskeleton were hardly detected.

FIG. 5B shows a graph illustrating the concentration of plasma creatinine (PCr). The concentration of the plasma creatinine increased with the increase in the renal injury.

FIG. 5C shows a graph illustrating the correlation between the amount of primary cilia and plasma creatinine (PCr). Due to the correlation between the excretion of primary cilia in urine and the increase of plasma creatinine, the change in the detected amount of primary cilia enables determination of the level of progress of a disease.

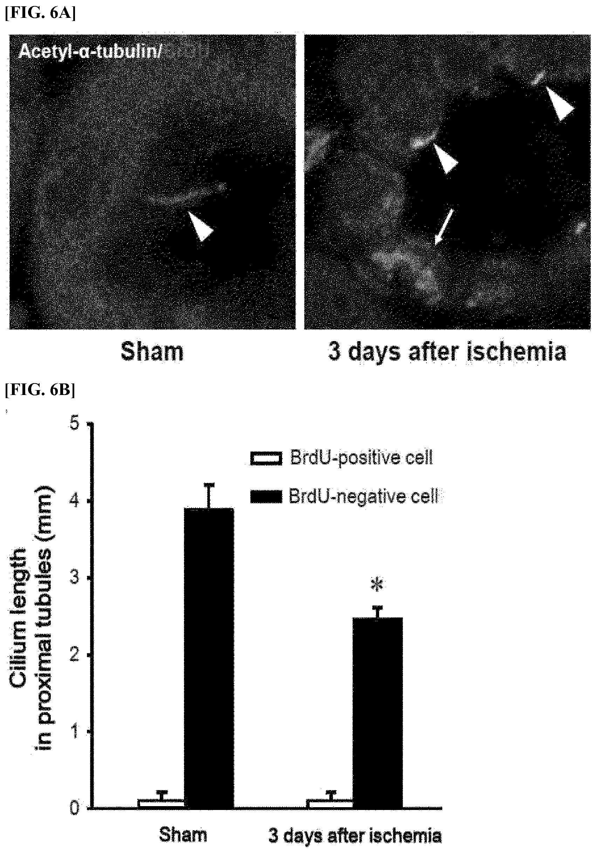

FIGS. 6A and 6B show the images and a graph illustrating the length of primary cilia in 5'-bromo-2'-deoxyuridine (BrdU) regenerating (incorporating) tubular epithelial cells (image A and graph B). Mice were subjected to bilateral renal ischemia for 30 minutes, and kidneys were harvested at a time-point after day 8. The mice were intraperitoneally administered with BrdU 20 hours prior to the harvest of the kidneys. The kidney sections were double-stained with anti-BrdU antibodies (red) and anti-acetylated α-tubulin antibodies (green). The nuclei were stained with DAPI (blue), and BrdU indicate regenerated cells.

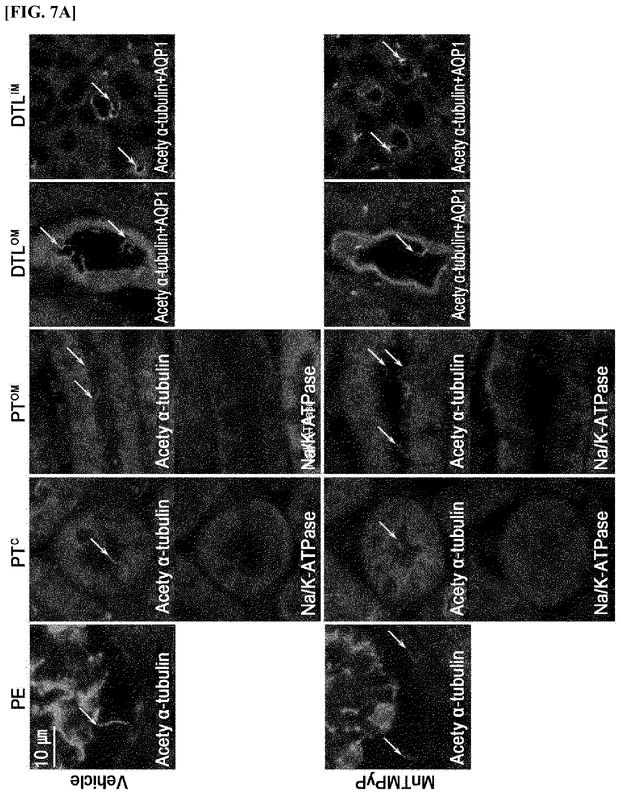

FIGS. 7A to 7C show images and a graph illustrating the effect of MnTMPyP treatment on the change in length of primary cilia after ischemia. Mice were subjected to bilateral renal ischemia for 30 minutes on day 0. Some mice were treated with MnTMPyP (MnT, 5 mg/kg body weight, peroxide production inhibitor) or saline daily starting from the 2^{nd} day of the operation. Eight days after the operation, the kidneys were harvested. (A and B) The kidney sections were double-stained with anti-acetylated α-tubulin antibodies (A, B) and other antibodies (anti-Na+-K+-ATPase antibodies (A, B), and anti-AQP-1 antibodies (A) or anti-AQP-2 antibodies (B)). The nuclei were stained with DAPI (blue). (C) The length of 30 primary cilia per kidney was measured and averaged. The results are expressed as mean ± SE (n=3). * p < 0.05 vs. vehicle.

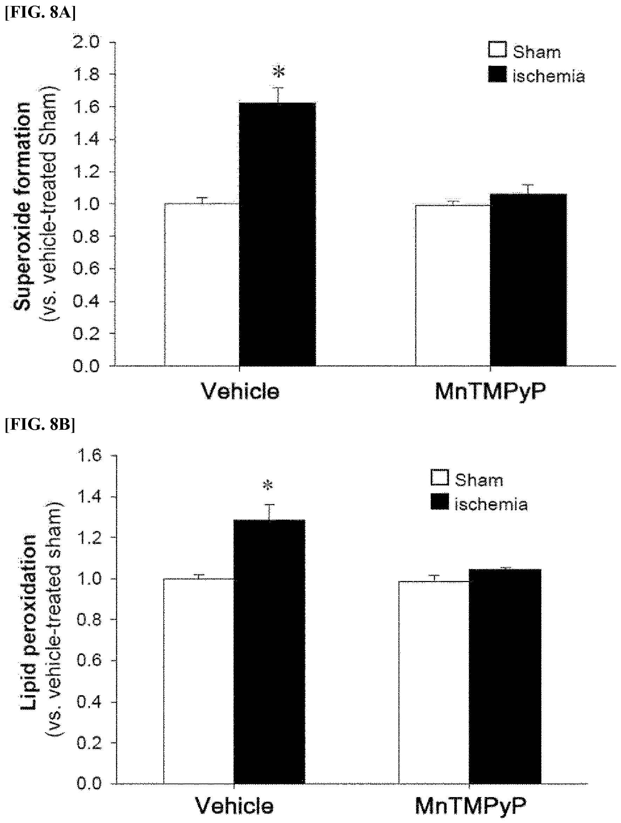

FIGS. 8A and 8B show graphs illustrating the effect of MnTMPyP treatment on peroxides (A) and lipid peroxidation (B) in the kidneys after ischemia/reperfusion injury. Mice were subjected to a bilateral renal ischemia or sham operation for 30 minutes on day 0. Some mice were treated with MnTMPyP (5 mg/kg body weight) or saline daily starting from the 2^{nd} day of the operation. Eight days after the operation, the kidneys were harvested, and peroxides and lipid peroxidation were measured. The results are expressed as mean ± SE (n=4). * P < 0.05 vs. sham.

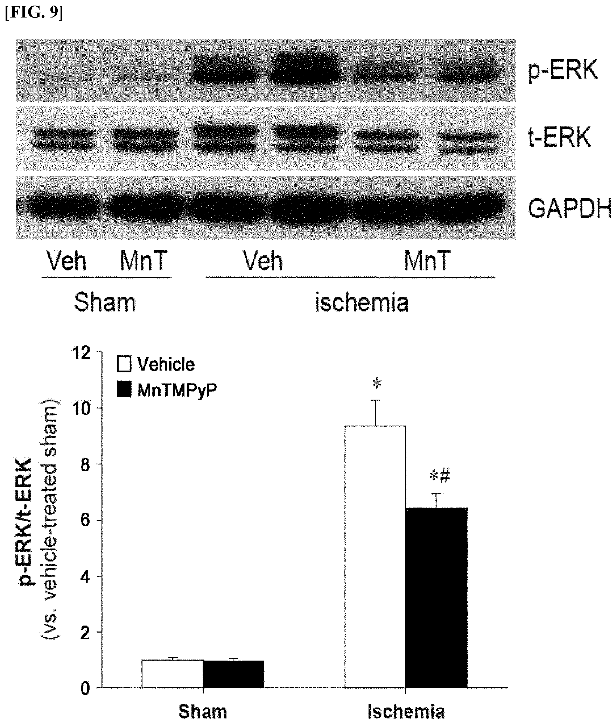

FIG. 9 shows a picture and a graph illustrating the effect of MnTMPyP treatment on ERK activation in the kidneys after ischemia/reperfusion injury. Mice were subjected to a bilateral renal ischemia or sham operation for 30 minutes on day 0. Some mice were treated with MnTMPyP (MnT, 5 mg/kg body weight) or saline daily starting from the 2^{nd} day of the operation. Eight days after the operation, the kidneys were harvested, and the levels of phosphorylated ERK and total ERK were determined. GAPDH expression was used as a control. Densities of blots were quantified using the Lab Works program. The measured values were expressed as mean ± SE (n=4).

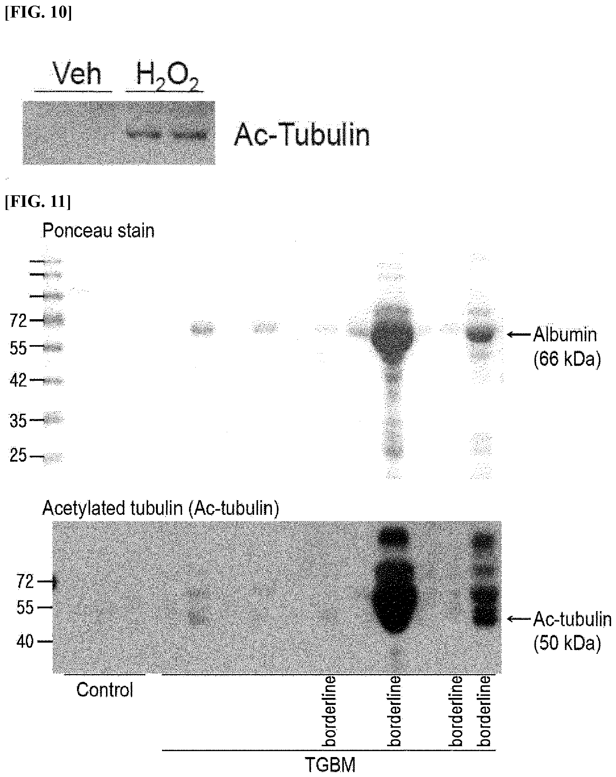

FIG. 10 shows the result of Western blot analysis confirming that primary cilia were isolated from cells into a medium after MDCK cells were induced with oxidative stress. After treating the renal tubule cells, MDCK, with 1 mM hydrogen peroxide for 4 hours, the cell medium was recovered and subjected to Western blot analysis using acetylated α-tubulin antibodies.

FIG. 11 shows the results of Western blot analysis confirming the detection of primary cilia fragments from the urine of a patient with thin glomerular basement membrane (TGBM) using acetylated α-tubulin antibodies.

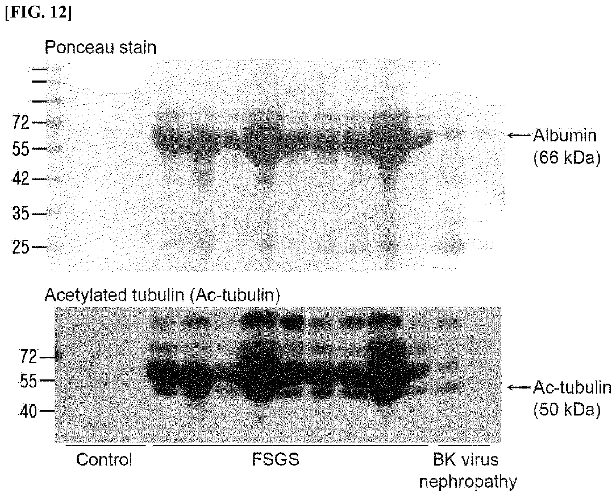

FIG. 12 shows the results of Western blot analysis confirming the detection of primary cilia fragments from the urine of a patient with focal segmental glomerulosclerosis (FSGS) or BK virus nephropathy using acetylated α-tubulin antibodies.

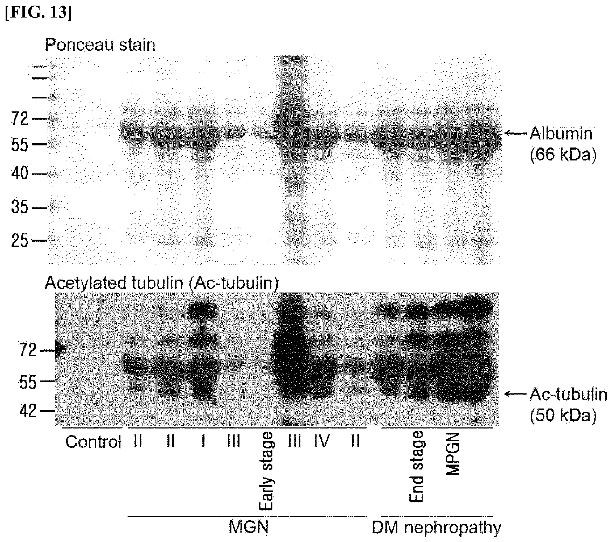

FIG. 13 shows the results of Western blot analysis confirming the detection of primary cilia fragments or a constituting protein thereof from the urine of a patient with membranous glomerulonephritis (MGN) or DM nephropathy using acetylated α-tubulin antibodies.

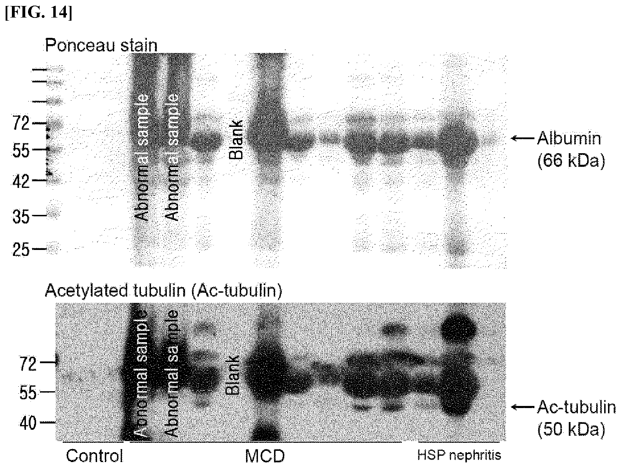

FIG. 14 shows the results of Western blot analysis confirming the detection of primary cilia fragments or a constituting protein thereof from the urine of a patient with minimal change disease (MCD) or Henoch-Schonlein Purpura (HSP) nephritis using acetylated α-tubulin antibodies.

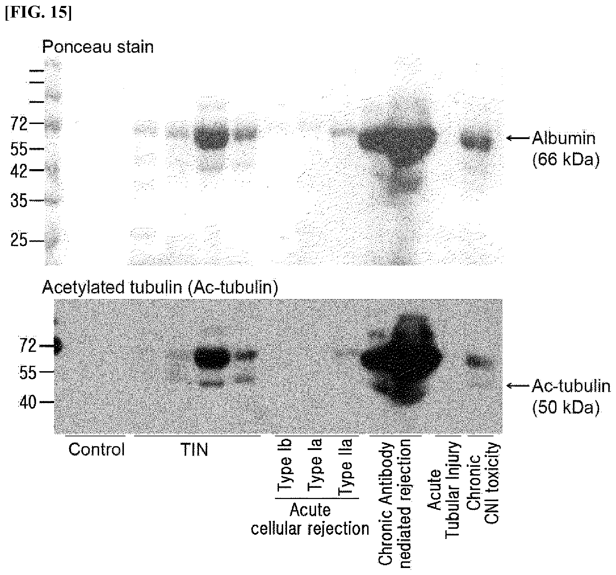

FIG. 15 shows the results of Western blot analysis confirming the detection of primary cilia fragments or a constituting protein thereof from the urine of a patient with tubulointerstitial nephritis (TIN), acute cellular rejection, chronic antibody-mediated rejection, chronic calcineurin inhibitor (CIN) toxicity, or acute tubular injury using acetylated α-tubulin antibodies.

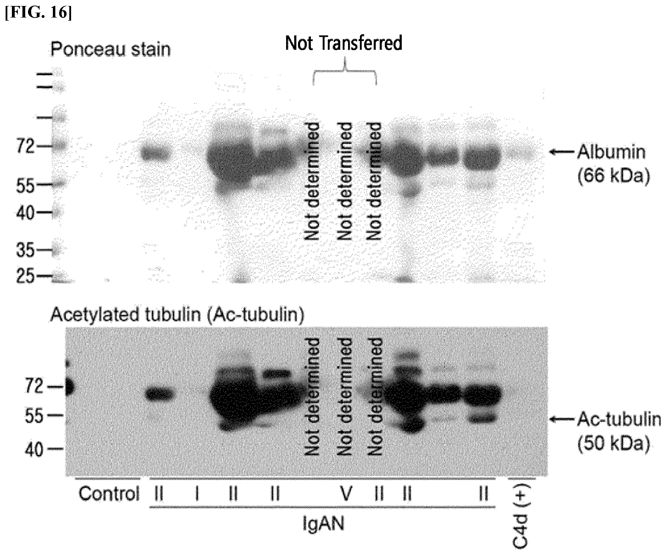

FIG. 16 shows the results of Western blot analysis confirming the detection of primary cilia fragments or a constituting protein thereof from the urine of a patient with IgA nephropathy (IgAN) or C4d using acetylated α-tubulin antibodies.

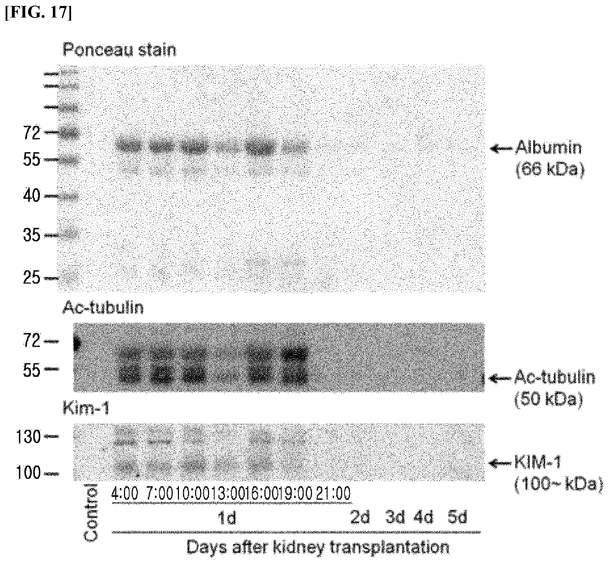

FIG. 17 shows the results of Western blot analysis confirming the detection of primary cilia fragments or a constituting protein thereof from the urine of a patient with kidney transplantation using acetylated α-tubulin antibodies.

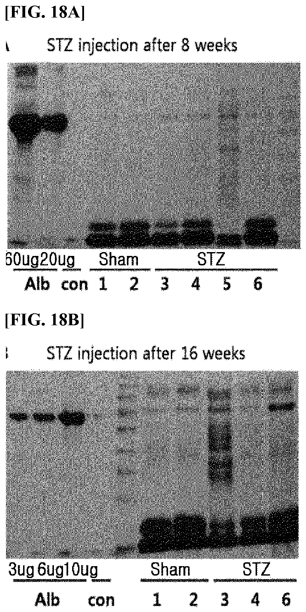

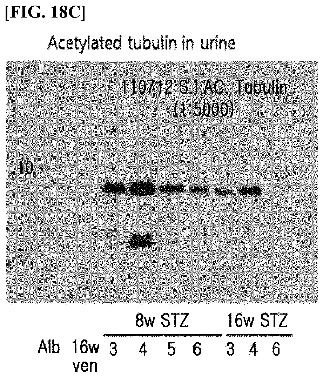

FIGS. 18A and 18B show the results confirming that the proteins were excreted into urine in a DM nephropathy-induced mouse by administering streptozotocin via electrophoresis of the urine followed by Coomassie staining, i.e., the occurrence of DM nephropathy. FIG. 18C shows the result of Western blot analysis confirming the detection of primary cilia fragments or a constituting protein thereof from the urine of the mouse using acetylated α-tubulin antibodies.

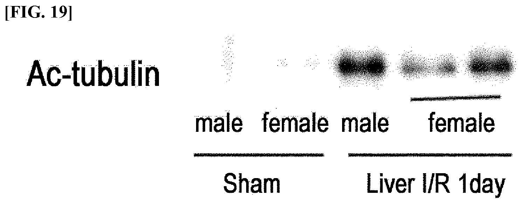

FIG. 19 shows the result of Western blot analysis confirming the detection of primary cilia fragments or a constituting protein thereof in the case of a renal injury induced by acute hepatic injury using acetylated α-tubulin antibodies.

### [BEST MODE]

In order to accomplish the above objects, in an aspect, the present disclosure provides a composition for urinalysis for diagnosing a renal disease containing an agent for specifically detecting primary cilia or a component thereof isolated from renal tissues, or basal bodies of the primary cilia or a component thereof isolated from renal tissues.

In the present invention, it was first verified that primary cilia or basal bodies of the primary cilia can be detected in a renal disease-specific manner in the urine, and the present inventors thereby completed the present invention for diagnosing a renal disease by detecting the primary cilia or basal bodies of the primary cilia isolated from renal tissues in the urine.

In the present invention, it was confirmed that the isolated primary cilia in patients with a renal disease and renal disease-induced mice were released into the urine, whereas the isolated primary cilia were not detected in the normal patients and normal mice. Accordingly, it was confirmed that the presence of a renal disease can be rapidly determined by detecting the presence of primary cilia or basal bodies of the primary cilia in the urine.

In particular, through consideration of the characteristics of the primary cilia, it was confirmed that the detection of primary cilia or basal bodies of the primary cilia in the urine is renal disease-specific. Specifically, primary cilia are present in many tissues other than renal tissues, however, the primary cilia in other tissues are not directly released into the urine even if they are isolated from cells.

For the release of the primary cilia isolated from tissues other than renal tissues into the urine, they should enter the blood stream through vascular walls, be filtered in the kidneys, and then released into the urethra after passing through the urinary bladder. However, it is physically impossible for the primary cilia to be passed through the vascular walls, and even if they are passed through the vascular walls, they cannot be excreted into the urine because they are either metabolized in the liver or they cannot pass through the glomerulus of the kidney. However, the primary cilia in the kidney extend to the surface of the lumen of renal tubules, and thus when the primary cilia are isolated they can be directly released into the urine. Accordingly, it was confirmed that the presence of a renal disease can be diagnosed with high specificity by detecting the presence of primary cilia or basal bodies of the primary cilia in the urine.

Additionally, the sample for the detection of the primary cilia, proteins constituting the same, or basal bodies of the primary cilia can be easily obtained, and also the detection procedure can be rapidly performed by a microscope or a reaction specific to the presence of the primary cilia (e.g., color development, immunochemical method, etc.). The detection of the primary cilia in the urine is easier than the detection in the tissues, and can be useful for the diagnosis of renal diseases.

Furthermore, in the present invention, it was confirmed that the presence of primary cilia, basal bodies of the primary cilia, and proteins constituting the same, and the contents thereof can be detected even with a small amount of urine of about 10 µL, and thereby it was confirmed that the diagnosis of renal diseases by detecting the presence of primary cilia in the urine has high sensitivity.

Additionally, as it was confirmed that primary cilia can be detected from the urine of patients and animals with various renal diseases in the present invention, the presence of primary cilia in the urine can be used not only for early detection of various renal diseases, but also for the prediction of degree of progress, prognosis, and prognosis after therapeutic treatment of renal diseases, according to the content of the primary cilia in the urine.

As used herein, the term "primary cilia or basal bodies of the primary cilia isolated from renal tissues" refers to those primary cilia or proteins constituting the primary cilia isolated from \kidneys due to a renal disease or structural damage to the primary cilia of renal tubules, and may also include basal bodies of the primary cilia and proteins constituting the same. Additionally, the primary cilia and the basal bodies of the primary cilia isolated from kidneys may be in the form of whole intact primary cilia or basal bodies thereof present in renal tissues, but may be a fragment (portion) thereof.

For the purposes of the present invention, the primary cilia or the basal bodies of the primary cilia isolated from renal tissues are cell structure materials which are mostly detected in the urine of a group having renal diseases or renal injuries. Since the primary cilia or the basal bodies of the primary cilia isolated from renal tissues are seldom present in the urine of the control group, the occurrence, degree of progress, and therapeutic effects and prognosis thereof of renal diseases may be diagnosed by detecting their presence and contents.

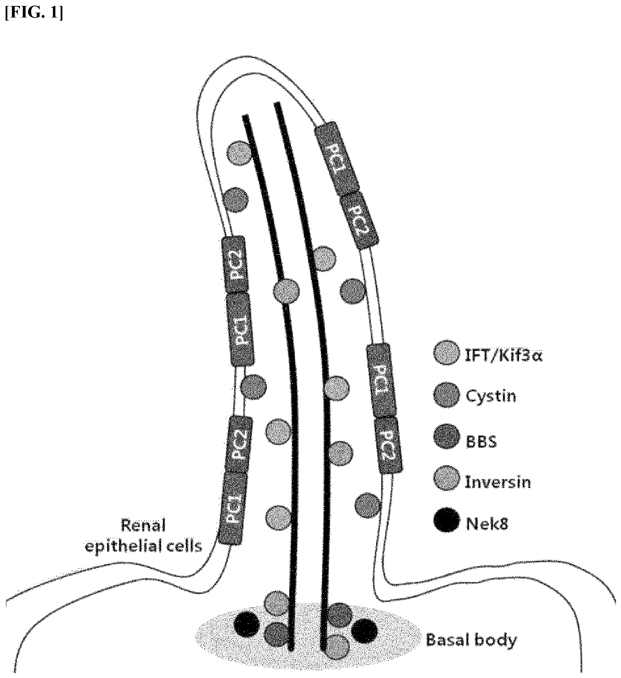

As used herein, the term "primary cilia" refers to microtubule-based non-motile intracellular organelles extending from the surface of most mammalian cells, including the epithelial cells of renal tubules. The cells in the medulla and the cortex that constitute kidneys have primary cilia on their surface. The structure of the primary cilia and the representative proteins constituting the same are illustrated in FIG. 1.

As used herein, the term "a constituting component of primary cilia isolated from renal tissues or a constituting component of basal bodies of the primary cilia isolated from renal tissues" representatively refers to a protein or non-peptide compound.

As used herein, the term "an agent for specifically detecting primary cilia isolated from kidneys or basal bodies of the primary cilia or a component thereof' may inclusively refer to an agent, which can detect their presence by an interaction with primary cilia isolated from kidneys or basal bodies of the primary cilia or the entire fragments thereof, or at least one component constituting the primary cilia or the basal bodies of the primary cilia.

The agent for specifically detecting the primary cilia isolated from kidneys or basal bodies of the primary cilia may also refer to an agent that can directly detect the proteins constituting the primary cilia or the basal bodies of the primary cilia thereon. Preferably, the agent is one that can bind to the proteins present, specifically to the primary cilia, to thereby detect their presence and measure their contents. In particular, the agent for specifically detecting the proteins, which constitute the primary cilia or the basal bodies of the primary cilia, is preferably one that can detect the proteins present specifically on the primary cilia or the basal bodies of the primary cilia.

The specific protein of the primary cilia in the methods of the invention is acetylated α-tubulin. Other disclosed examples of the proteins specifically present in the primary cilia may include EVC2, INPP5E, INVERSIN, NPHP3, Broad-Minded (bromi), FAM49B, GTL3, TSGA14, WDR60, WDR11, polycystin-1, polycystin-2, NME7, SEPT2, SEPT7, SEPT9, small GTPase Ran, importin, IFT57, IFT74, IFT80, IFT88, IFT122, IFT172, etc., but any protein which, being a primary cilia-constituting protein, is hardly present in urine other than in the forms of primary cilia or a fragment thereof and is thus able to distinguish a normal person from a patient with a renal disease, may be applicable for the specific detection of primary cilia in the present invention. In an exemplary embodiment of the present invention, antibodies to the acetylated α-tubulin, which is the representative protein present in primary cilia, were used.

As used herein, the term "acetylated α-tubulin" refers to a kind of protein constituting the primary cilia. For the purposes of the present invention, the presence of acetylated α-tubulin, being a protein excessively contained in the urine of renal disease patients, may be detected from the urine, and the presence or amount of primary cilia, primary cilia fragments, and proteins constituting the primary cilia may be measured to thereby diagnose renal diseases.

Preferably, the agent for specifically detecting the basal bodies of primary cilia isolated from kidneys is one that can bind to proteins constituting the basal bodies of the primary cilia. The basal body is a region of a cell that is directly connected to the primary cilia, and it may be released from the cell when the primary cilia are isolated and thus can be detected in the urine. The specific protein of the basal bodies of the primary cilia in the methods of the invention is ARL13B. Other disclosed examples of the basal body may include tektins, pf-ribbon, CEP164, ODF2, CEP170, etc., but any protein which, being a protein constituting the basal bodies of primary cilia, is hardly present in the urine other than in the form of the basal bodies of primary cilia and thus enables distinguishing of normal people from the group of people with renal diseases, may be sufficient. In an exemplary embodiment of the present invention, the antibodies to Arl13b were used.

Because the basal bodies of primary cilia are detected when the disease has progressed further, compared to the detection of the primary cilia, the investigation of the quantitative change in the basal bodies and the primary cilia can be used for determining the intensity, progress, etc., of the disease. Accordingly, the composition for urinalysis of the present invention may include both the agent for specifically detecting the primary cilia isolated from renal tissues or a component thereof, and the agent for specifically detecting the basal bodies of primary cilia isolated from renal tissues or a component thereof.

Non-limiting examples of the primary cilia or the basal bodies of primary cilia or a component thereof isolated from kidneys may include antibodies, aptamers, etc., but are not particularly limited thereto.

As used herein, the term "antibody" refers to a proteinaceous molecule that can bind specifically to an antigenic region of a protein or peptide molecule. The antibody may not be limited to having a particular form, but may include all immunoglobulin antibodies as long as it has a binding activity to polyclonal antibodies, monoclonal antibodies, or antigens, and may also include particular antibodies such as humanized antibodies, etc. Additionally, the antibody includes not only the complete form having two full-length light chains and two full-length heavy chains, but also functional fragments of antibody molecules. The functional fragment refers to a fragment having at least the antigen-binding ability, and may be Fab, F(ab'), F(ab')₂, Fv, etc.

As used herein, the term "aptamer" refers to a single-stranded nucleic acid (DNA, RNA, or modified nucleic acid) having the characteristics of being capable of binding to a target molecule with high affinity and specificity while having a stable tertiary structure itself. For the purposes of the present invention, the aptamer refers to an aptamer which can bind to at least one protein constituting primary cilia or a fragment thereof, but is not limited thereto.

The composition for urinalysis according to the method of the present invention, in order to improve detection efficiency of primary cilia or basal bodies thereof derived from renal tissues, may further contain a treating agent for decomposing the primary cilia or the basal bodies thereof into the proteins constituting the same. The agent for protein extraction may not be particularly limited as long as it can extract the proteins constituting the primary cilia or the basal bodies thereof from the same. Preferably, the agent may be a protease that can exhibit an activity to randomly decompose proteins but not to the level to decompose them into amino acids; or a surfactant, etc., which can decompose the bond between proteins while not being involved in the peptide bond constituting the proteins, more preferably, the agent may be a protease such as trypsin, chymotrypsin, plasmin, lysozyme, etc., or a surfactant such as NP-40, SDS, Triton X-100, SLS, etc., and most preferably, a surfactant such as NP-40, SDS, Triton X-100, SLS, etc.

As used herein, the term "renal disease" refers to a disease occurring in kidneys due to various reasons including extrinsic factors, intrinsic factors, genetic factors, etc. Examples of the renal disease may include thin glomerular basement membrane (TGBM), minimal change disease (MCD), membranous glomerulonephritis (MGN), focal segmental glomerulosclerosis (FSGS), DM nephropathy, IgA nephropathy (IgAN), tubulointerstitial nephritis (TIN), Henoch-Schonlein Purpura (HSP) nephritis, acute tubular injury, BK virus nephropathy, acute cellular rejection, chronic antibody mediated rejection, chronic active antibody mediated rejection, chronic calcineurin inhibitor toxicity, acute kidney injury, ischemic renal disease, glomerulonephritis, lupus nephritis, polycystic kidney disease, pyelonephritis, nephrolith, renal tuberculosis, renal tumor, chronic renal failure, end stage renal failure, sepsis, renal injury caused by hepatic injury, etc., but are not limited thereto, and may include, without limitation, any disease in which primary cilia or basal body of primary cilia can be detected in the urine when the disease occurs. A serious injury on an organ located far from the kidneys may cause an injury on the kidneys, and in this case, primary cilia may be detected in the urine.

As used herein, the term "diagnosis" refers to confirming the presence of a pathological state, a progress of a disease, therapeutic effects on a disease when treated, etc. For the purposes of the present invention, the diagnosis refers to diagnosing a renal disease by detecting primary cilia, a basal body thereof, or a structure material constituting the same (e.g., proteins), but is not limited thereto.

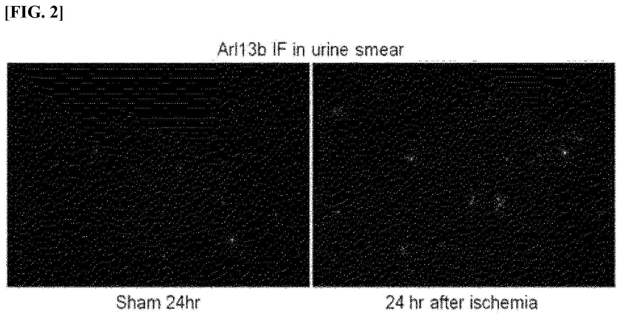

In an exemplary embodiment of the present invention, it was confirmed that 30 minutes of bilateral renal ischemia can cause renal injury based on the observations of injuries on renal tubule cells and the increase in plasma creatinine concentration (FIG. 3). Additionally, basal bodies of primary cilia were detected in the urine of mice with renal injuries induced by the ischemia described above by observing under a microscope (FIG. 2). Furthermore, it was confirmed that the primary cilia are present in the urine of the ischemia-induced group while they are not present in the urine of the normal control group using acetylated α-tubulin, which is a primary cilia-constituting component (FIG. 5), and thereby it was confirmed that the primary cilia, basal bodies of the primary cilia, or proteins constituting the same can be used as specific markers for renal diseases. Additionally, it was confirmed that acetylated α-tubulin was present according to the ischemia-inducing time (the disease becomes severe as the ischemia-inducing time becomes longer), that is, the disease was present in an intensity-dependent manner. Furthermore, arl13b, a protein constituting the basal part of the primary cilia, was also detected, thus confirming that an agent capable of detecting the proteins constituting the basal part of the primary cilia can be also used in the composition for urinalysis for diagnosing renal diseases (FIG. 2). Since the primary cilia were detected in the urine more rapidly than the basal bodies of the primary cilia, it was confirmed that the presence/absence of the primary cilia and the basal bodies of the primary cilia in the urine can determine the progress of a renal disease (FIG. 2). In particular, it was confirmed that the primary cilia can be detected in all urine samples of patients with various renal diseases such as thin glomerular basement membrane (TGBM), minimal change disease (MCD), membranous glomerulonephritis (MGN), focal segmental glomerulosclerosis (FSGS), DM nephropathy, IgA nephropathy (IgAN), tubulointerstitial nephritis (TIN), Henoch-Schonlein Purpura (HSP) nephritis, acute tubular injury, BK virus nephropathy, acute cellular rejection, chronic antibody mediated rejection, acute renal injury after kidney transplantation, injury on transplanted kidneys, and chronic calcineurin inhibitor (CIN) toxicity (FIGS. 11 to 17). Additionally, fragments of the primary cilia were also confirmed to be detected in mice with type I DM neuropathy and in mice induced with acute hepatic disease, thus suggesting that the primary cilia or basal bodies of the primary cilia can be used in the diagnosis of various renal diseases (FIGS. 18 and 19).

In another aspect, the present disclosure provides a kit for urinalysis for diagnosing a renal disease, including a substrate for adsorbing to or receiving primary cilia or a component thereof isolated from renal tissues, or basal bodies of the primary cilia or a component thereof isolated from renal tissues; or isolating primary cilia or basal bodies of the primary cilia isolated from renal tissues from urine.

For the increase of detection sensitivity, the urine may be subjected to centrifugation, concentration, etc.

The primary cilia, basal bodies of the primary cilia isolated from renal tissues, the component thereof, and renal diseases are the same as described above.

The substrate used for the kit of the present disclosure may further contain a chromophore for confirming the presence of a contact when the substrate is brought into contact with urine.

To improve sensitivity and specificity, the kit may be used along with a conventional kit for diagnosing renal diseases, for example, one used in the form of a combined kit.

Specifically, renal diseases can be diagnosed using the kit by detecting, in the urine sample, the primary cilia or the basal bodies thereof or the proteins constituting the same using a substrate capable of being adsorbed to or receiving the primary cilia isolated from renal tissues or a component thereof, or basal bodies of the primary cilia isolated from renal tissues or a component thereof. Additionally, renal diseases can be diagnosed by confirming the presence and contents of the primary cilia or basal bodies thereof using a substrate which can separate the primary cilia or basal bodies thereof from other components in the urine sample.

In particular, the substrate for isolating the primary cilia or basal bodies thereof may be a sieve for sifting the primary cilia or basal bodies thereof, however, the substrate is not particularly limited as long as it can isolate the primary cilia or basal bodies thereof from other urine samples. The mesh of the sieve is one that can isolate the primary cilia or basal bodies thereof, and may be appropriately selected by one of ordinary skill in the art. The thus-isolated primary cilia or basal bodies thereof may be confirmed by a microscope, the naked eye, a color-developing material, etc., but is not limited thereto.

In the above kit, the types of the substrate to be adsorbed to or received into the proteins constituting the primary cilia isolated from the renal tissues or basal bodies thereof or proteins constituting the same are not particularly limited, and urine-insoluble materials such as papers, polymer substrate, glass, etc., may be used. The primary cilia or basal bodies thereof or proteins constituting the same may be confirmed using a microscope, the naked eye, a color-developing material, the composition of the present invention, etc., but are not limited thereto.

In order to confirm the presence or concentration of the primary cilia isolated from renal tissues, basal bodies thereof, or proteins constituting the same, urine samples may be first treated with a color-developing agent or detecting agent and then with the substrate in the kit for urinalysis.

The kit may include an agent for specifically detecting the primary cilia isolated from renal tissues or a component thereof, or basal bodies of the primary cilia or a component thereof.

Examples of the agent for confirming the presence and length of the primary cilia may include dyes that can bind to the primary cilia. That is, the presence and length of the primary cilia may be confirmed by the dye bound thereto.

In the kit, the detection agent (e.g., antibodies or fluorescent antibodies) or the color-developing agent (e.g., dyes) may be received into or adsorbed to the substrate being dissolved in the urine. When the substrate is dipped into a urine sample, the substrate becomes dissolved, thereby releasing the detection agent or the color-developing agent contained therein into the urine sample. The released agent(s) can bind to or react with the primary cilia or basal bodies thereof or components thereof, and from these, the presence and content of the primary cilia or basal bodies thereof or components thereof can be confirmed and quantified via the color, absorbance, or degree of fluorescence of the urine.

The detection agent may also be contained in the substrate to adsorb to or receive the primary cilia isolated from renal tissues or basal bodies thereof, or may be present separately from the substrate for adsorption or isolation.

The detection agent contained in the substrate may specifically bind to the primary cilia or basal bodies thereof isolated from renal tissues or proteins constituting the same, thereby playing the role of specifically isolating and/or detecting only the primary cilia or fragments thereof, either by allowing other materials in the urine to pass by or by not reacting with them.

Additionally, the detection agent may be an agent for measuring the level of the proteins.

As used herein, the term "an agent for measuring the level of proteins" refers to an agent used in the method for measuring the level of a target protein contained in the sample. Examples of the agent may include antibodies used in the methods of Western blotting, enzyme linked immunosorbent assay (ELISA), radioimmunoassay (RIA), radioimmunodiffusion, Ouchterlony immunodiffusion, rocket immunoelectrophoresis, histoimmuno staining, immunoprecipitation assay, complement fixation assay, FACS, protein chip assay, etc., or aptamers or the like that can bind to the proteins, but is not particularly limited thereto.

The kit for measuring the expression level of the proteins constituting the primary cilia may include, for the immunological detection of antibodies, a substrate, an appropriate buffer, a secondary antibody labeled with a chromogenic enzyme, or a color-developing substrate, etc. Examples of the substrate may include a nitrocellulose membrane, a 96-well plate synthesized by polyvinyl resin, a 96-well plate synthesized by polystyrene resin, a slide glass made of glass, etc. Examples of the chromogenic enzyme may include peroxidase, alkaline phosphatase, etc.; examples of the fluorescent material may include FITC, RITC, etc.; and examples of the color-developing substrate may include 2,2'-azino-bis-(3-ethylbenzothiazoline-6-sulfonic acid) (ABTS), o-phenylenediamine (OPD), or tetramethylbenzidine (TMB).

Additionally, the kit may further include secondary antibodies which can detect the above antibodies or aptamers, and those labeled with fluorescent materials, radioisotopes, etc., may be used.

The kit is advantageous in that it can diagnose renal diseases by detecting the primary cilia from the urine in an amount of 1 µL to 50 µL, and preferably in a small amount of 10 µL.

The kit may further include a treating agent for decomposing the primary cilia isolated from renal tissues or basal bodies thereof into proteins constituting the same.

In another aspect, the present disclosure provides a method for detecting primary cilia or a component thereof isolated from renal tissues, or a primary cilia support or a component thereof isolated from renal tissues, including confirming the presence of the primary cilia or a component thereof isolated from renal tissues, or the primary cilia support or a component thereof isolated from renal tissues, from an isolated urine sample.

The primary cilia, basal bodies thereof, and components constituting the same are the same as described above.

The presence of the primary cilia, basal bodies thereof, and/or components constituting the same in the separated urine may be identified using an agent for specifically detecting the same, or a substrate isolating the primary cilia or basal bodies thereof, dyes binding to the primary cilia or basal bodies thereof, etc., but is not limited thereto.

Prior to the step above, the method may further include treating the separated urine with a treating agent in order to decompose the primary cilia into proteins constituting the primary cilia.

In another aspect, the present invention provides a method for diagnosing the occurrence or progress of a renal disease, including confirming the presence of primary cilia or a component thereof isolated from renal tissues, or basal bodies of the primary cilia or a component thereof isolated from renal tissues; the concentrations thereof; or the length of the primary cilia thereof, from an isolated urine sample.

The primary cilia, basal bodies thereof, and components constituting the same are the same as described above.

Additionally, the method for diagnosing the occurrence or progress of renal diseases can also be used to confirm the therapeutic effects on renal diseases and the effect of prognosis.

Specifically, the method can confirm the presence of the primary cilia, basal bodies thereof, or acetylated α-tubulin or Arl13b, in isolated urine. When the primary cilia, basal bodies thereof, or acetylated α-tubulin or Arl13b is present in the separated urine, it can be determined that there is a disorder in kidneys and the occurrence of a renal disease. Additionally, (a) detecting the primary cilia or basal bodies thereof from the separated urine of a normal person and the separated urine of a subject to be detected; and (b) comparing the level of the primary cilia or basal bodies thereof detected from the separated urine of a normal person with that of the separated urine of a subject to be detected, and confirming that the level of the primary cilia or basal bodies thereof detected from the separated urine of the normal person shows a significant increase compared to that of the separated urine of the subject to be detected, it can be determined that a renal disease occurred in the subject.

Additionally, by measuring the content, concentration, or length of the primary cilia, basal bodies thereof, or components constituting the same (e.g., acetylated α-tubulin or Arl13b) from the isolated urine sample, the progress of renal diseases can also be determined according to the degree of increase in content and the concentration of proteins constituting the same (FIG. 5).

In particular, the primary cilia or basal bodies thereof can be detected by various methods, including a method of confirming primary cilia or basal bodies thereof contained in a urine sample by applying the urine sample directly to means such as a microscope, a method of confirming primary cilia or fragments thereof in a urine sample using means such as a microscope after filtration and concentration, and a method of confirming the markers for primary cilia or basal bodies thereof at the protein level. The method of confirming the markers at the protein level is the same as explained above.

The step of confirming may refer to confirming the presence, concentration, or length of the primary cilia by treating the primary cilia or basal bodies thereof isolated from renal tissues with an agent that can specifically detect the same or with an agent that can specifically detect the components thereof (e.g., acetylated α-tubulin or Arl13b).

The method of diagnosis according to the present invention may further include, prior to the step above, treating the isolated urine sample with an agent to decompose the primary cilia into components constituting the same.

Additionally, the present invention enables diagnosis of the severity or progress of diseases by investigating the quantitative change in the primary cilia and the basal bodies of the primary cilia.

### [DETAILED DESCRIPTION OF THE INVENTION]

Hereinafter, the present invention will be described in more detail with reference to the following Examples. However, these Examples are for illustrative purposes only, and the invention is not intended to be limited by these Examples.

### Example 1: Materials and methods of experiment

### (1) Experimental animals

Experiments were performed using 8-week-old C57B/6 mice, and the mice were anesthetized with an anesthetic (pentobarbital sodium; 60 mg/kg of body weight) prior to an ischemia/reperfusion or sham operation. Renal ischemia was performed according to a known method by the occlusion of renal pedicles using nontraumatic microaneurysm clamps (Roboz, Rockville, MD) for 30 minutes (Park KM, Chen A, and Bonventre JV., The Journal of biological chemistry 276: 11870-11876, 2001). Cohorts of the mice were intraperitoneally administered daily with Mn(III) tetrakis(1-methyl-4-pyridyl) porphyrin (5 mg/kg of body weight), which is an antioxidant capable of exhibiting the effect of superoxide dismutase (SOD), and 0.9% saline beginning 48 hours after ischemia until 24 hours before the experiment. Additionally, some of these mice were administered intraperitoneally with 5'-bromo-2'-deoxyuridine (BrdU, 50 mg/kg of body weight) 20 hours before the experiment. In order to induce DM nephropathy, the mice were administered once daily with streptozotocin (55 mg/kg of body weight) for 5 days, and the blood sugar level was confirmed by measuring the glucose level in the blood. The occurrence of DM nephropathy by induction was confirmed by observing the presence of a protein in the urine, i.e., proteinuria, by Coomassie Blue staining. The presence of the primary cilia in the urine was detected by Western blot analysis using acetylated tubulin antibodies.

In order to induce acute renal injury caused by acute hepatic injury, the hepatic artery and the hepatic vein of the mice were ligated, the urine was collected 24 hours thereafter, and the presence of the primary cilia in the urine was detected by Western blot analysis using the acetylated tubulin antibodies.

### (2) Histological analysis

Kidneys were perfused via the left ventricles with 30 mL of PBS for 2 minutes and then perfused with PLP solution (2% paraformaldehyde, 75 mM L-lysine, and 10 mM sodium metaperiodate). Then, the kidneys were ablated and immersed into the PLP solution at 4°C for 4 hours. Paraffin-embedded tissue samples were sliced into 4 µm-thick sections using a microtome, and the paraffin sections were stained with periodic acid Schiff (PAS) to determine histological damage. The PAS staining was performed according to the manufacturer's protocols. Images were collected after photographing using a digital camera. Each experimental group consisted of more than four mice.

### (3) Renal functional parameters

The concentration of plasma creatinine (PCr) was measured using a Beckman Creatinine Analyzer II (Beckman, Brea, CA).

### (4) Immunofluorescence assay

Paraffin-embedded tissue sections were deparaffinized with xylene, rehydrated with 100%, 95%, and 80% ethanol in this order, and washed with PBS for 10 minutes, respectively. The sections were immersed in PBS containing 0.1% SDS for 5 minutes and then washed with PBS for 10 minutes. To reveal the antigen epitopes, the sections were boiled in 10 mM sodium citrate buffer (pH 6.0) for 10 minutes in an autoclave, cooled at room temperature for 20 minutes, and washed three times with PBS for 5 minutes, respectively. The sections were blocked by immersing in PBS containing 1% bovine serum albumin (BSA) at room temperature for 30 minutes, and then reacted overnight at 4°C with the respective antibodies (anti-acetylated α-tubulin antibodies (Ac-tubulin, 1:100; Sigma), anti-Na/K-ATPase antibodies (1:20 dilution; Santa Cruz, CA), anti-BrdU antibodies (1:100 dilution; Serotec, Raleigh, NC), anti-aquaporin-1 antibodies (AQP1; 1:100 dilution; Alomone Labs, Jerusalem, Israel), and anti-AQP2 antibodies (1:200 dilution; Alomone Labs)). Upon completion of the reactions, each of the sections was washed three times with PBS for 5 minutes, reacted with FITC- or Texas Red-conjugated secondary antibodies at room temperature for 60 minutes, and washed three times with PBS for 5 minutes, respectively. To detect the cell nuclei, 4'-6-diamidino-2-phenylindole (DAPI) was applied to each of the sections for 1 minute, respectively. Finally, the sections were mounted on Prolong Gold anti-fade reagent (Invitrogen, Carlsbad, CA) and observed under an Axioplan-2 epifluorescence microscope. Each experimental animal group consisted of three mice.

### (5) Measurement of levels of superoxide formation and lipid peroxidation in renal tissues

The superoxide level in renal tissues was measured using dihydroethidium (DHE) in a fluorescence spectrometer (Molecular Devices). In brief, the kidneys ablated from mice were homogenized in ice to obtain the kidney lysates. Then, 200 µL of 10 µM DHE was added to 96-well plates, to which 20 µL of the kidney lysates was aliquoted. The fluorescence emitted from the plates was read at 37°C using an emission/excitation filter. Superoxide levels were expressed as the value per milligram protein of kidney lysates. The level of lipid peroxidation was measured using thiobarbituric acid-reactive substances (TBARS).

### (6) Western blot analysis

Western blot analyses were performed according to a method described in Park KM (Park KM, Chen A, and Bonventre JV., The Journal of biological chemistry 276: 11870-11876, 2001), etc. In brief, renal tissue lysates were separated on 10% SDS-PAGE gels and then transferred to Immobilon membranes. The membranes were reacted with anti-p-ERK antibodies, anti-total ERK antibodies, anti-CPI-17 antibodies, anti-beta-actin antibodies, anti-KIM-1 (kidney injury molecule-1) antibodies, and anti-GAPDH antibodies. Upon washing, the membranes were reacted with the peroxidase-conjugated secondary antibodies against the primary antibodies, exposed to Western Lighting Chemiluminescence Reagent (NEL101), and the developed using X-ray films.

### (7) Detection of primary cilia fragments in urine samples of mice

Urine samples were collected from the mice subjected to a sham or ischemia/reperfusion operation using a metabolic cage. An SDS sample buffer was added to each of the urine samples of 10 µL and heated at 98°C for 5 minutes. Western blot analyses were performed using anti-acetylated tubulin antibodies, which can detect primary cilia fragments, according to the method described above.

### (8) Detection of cilia fragments in cell culture after hydrogen peroxide treatment

A liquid cell culture in the amount of 10% was collected from the control group or the MDCK cells (distal renal tubule cells) treated with hydrogen peroxide. The analysis of the acetylated tubulin was performed for the SDS samples prepared using the liquid cell culture according to the method described above.

### (9) Confirmation of occurrence of diabetic nephritis and detection of primary cilia in urine of mouse showing type I diabetic nephritis by administration of streptozotocin

Mice were administered with streptozotocin (55 mg/kg of body weight) for 5 days. Then, the urine of the mice at 8 weeks and 16 weeks thereafter was collected. The SDS samples prepared from the collected urine were subjected to Coomassie Blue staining to analyze the presence of proteinuria, and the excretion of acetylated tubulin protein into the urine was examined using the anti-acetylated tubulin antibodies.

### (10) Confirmation of occurrence of diabetic nephritis and detection of primary cilia in urine of mouse with acute hepatic injury

In order to detect the presence of primary cilia fragments in the urine of a mouse with hepatic injury induced by ischemia/reperfusion, the hepatic artery and the hepatic vein of the mouse were simultaneously occluded to induce a hepatic injury, and the urine of the mouse was collected 24 hours thereafter to prepare a urine SDS sample, and the excretion of the acetylated tubulin protein into the urine was examined using the acetylated tubulin antibodies.

### (11) Statistical analysis

Each of the experimental results was expressed as mean ± SE. Statistical differences among different groups were calculated via analysis of variance (ANOVA) and post hoc comparison using SPSS 12.0 program. Differences among groups were considered statistically significant at a *P* value of < 0.05.

### Example 2: Changes in renal functions and histology after Ischemia/Reperfusion (I/R) injury

In this example, it was examined whether a renal injury induced by ischemia/reperfusion may result in changes in the functions and histological characteristics of kidneys.

Specifically, at the time-point of 24 hours after ischemia, the concentration of plasma creatinine increased rapidly and then returned to normal 8 days and 16 days after the ischemia (FIG. 3A).

At the time-point of 24 hours after ischemia, the collapse, dilation, thinning, shrinkage of tubules, deposition of impurities within tubules, and damage to tubule cells appeared (FIG. 3B). It was confirmed that the damage to the proximal tubules of the outer medulla was more serious than to the tubules present in other regions (FIG. 3B).

At the time-points of 8 days and 16 days after ischemia, the number of shrunken or dilated tubules was decreased compared to that observed at the time-point of 24 hours after ischemia (FIG. 3B). This result indicates that a histological restoration occurred in the kidney with ischemia/reperfusion injury.

However, it was confirmed that a complete histological restoration was not achieved until 16 days after the ischemia/reperfusion injury (FIG. 3B).

### Example 3: Change in primary cilia length after ischemia

The presence of primary cilia and the change in their length after ischemia were observed at a tissue level.

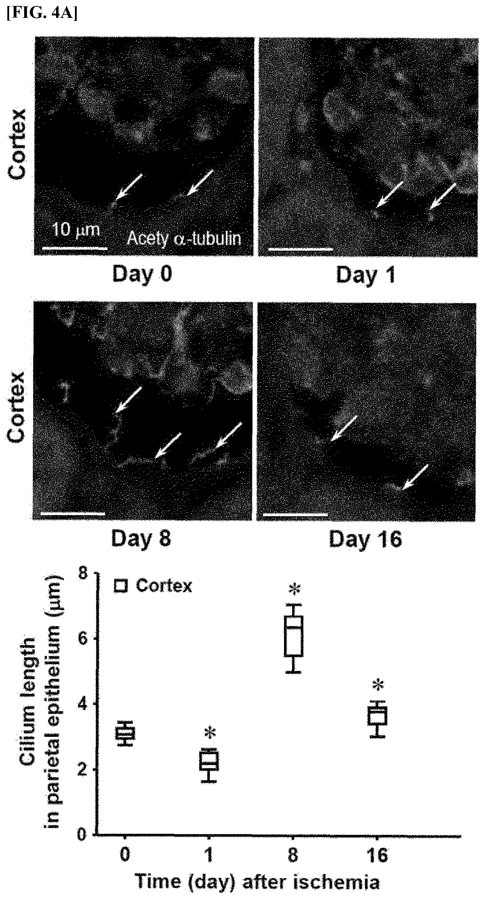

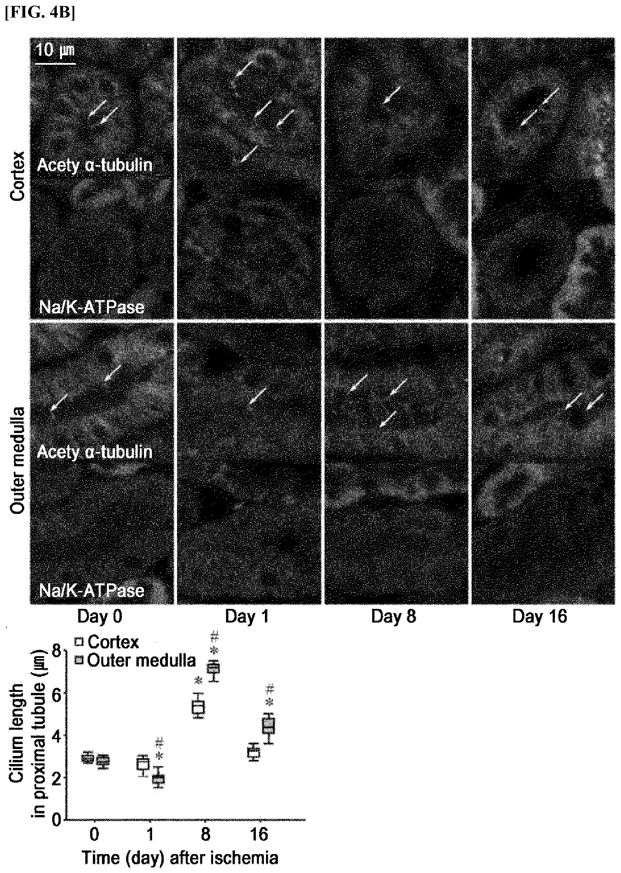

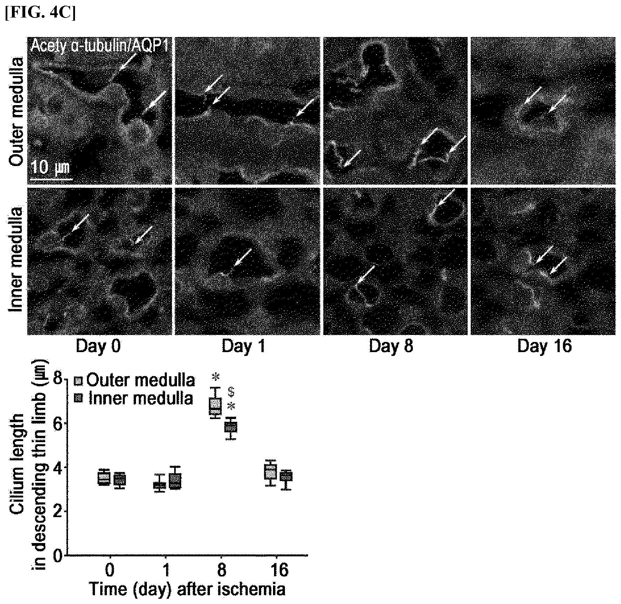

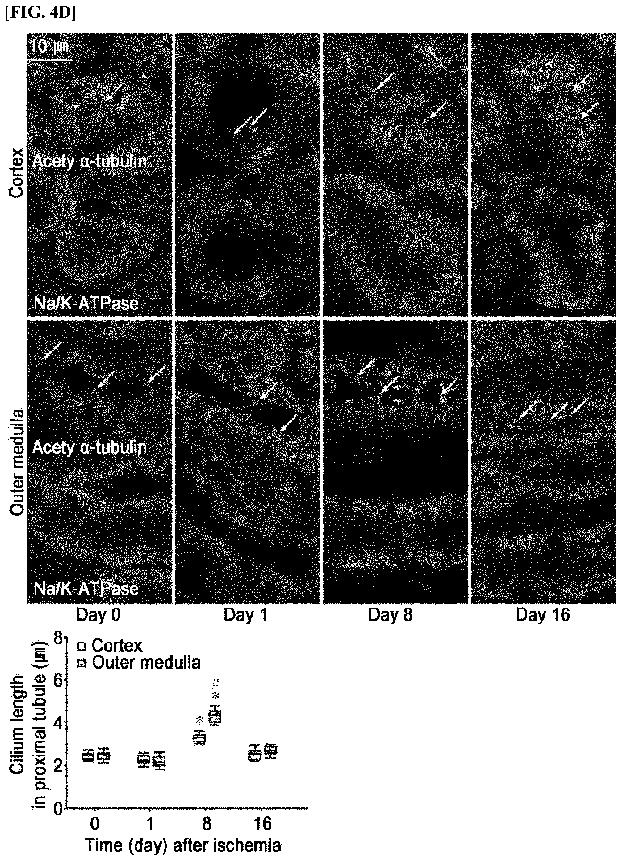

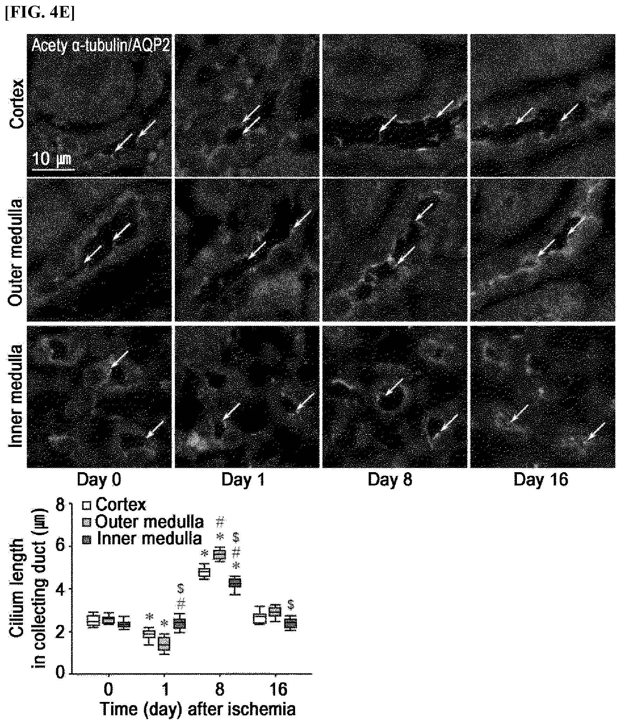

As a result, as shown in FIG. 4, the primary cilia were found in the renal tubular epithelial cells and parietal cells at normal state, and the primary cilia were observed in all tubules as is known, however, the primary cilia were not found in interstitial cells of collecting ducts (FIG. 4).

When the change in the length of the primary cilia was examined from the parietal layer of Bowman's capsule, as compared to the control group which was subjected to a sham operation, the length of the primary cilia was shorter at the time-point of 1 day after the ischemia/reperfusion injury, however, at the time-point of 8 days after the ischemia/reperfusion injury, the length of the primary cilia was elongated, and at the time-point of 16 days after the ischemia/reperfusion injury, the length of the primary cilia returned to a similar level to that of the normal control (FIG. 4A).

The length of the primary cilia of the proximal tubules was measured at the time-point of 1 day after the ischemia/reperfusion injury, and the results are shown in FIG. 4B. According to the results, the length of the primary cilia in the outer medulla, unlike in the cortex, was shortened compared to that of the sham-operated control cells. This indicates that the primary cilia of the proximal tubular epithelial cells in the outer medulla were more susceptible to the ischemia/reperfusion injury compared to those present in the cortex. Additionally, the proximal tubular epithelial cells in the outer medulla showed a serious collapse of Na/K-ATPase. At the time-point of 8 days after ischemia, the lengths of the primary cilia in the proximal tubule cells both in the cortex and outer medulla were significantly elongated, compared to those in the sham-operated kidneys. At the time-point of 16 days after ischemia, the lengths of the primary cilia in the proximal tubular epithelial cells in the cortex, but not in the outer medulla, returned to near normal ranges (FIG. 4B).

Meanwhile, the presence and length of primary cilia in the cells of the descending thin limbs in the inner and outer medulla were observed and the results are shown in FIG. 4C. According to the results, there was no significant change at the time-point of 1 day after ischemia injury, however, at the time-point of 8 days after ischemia, the length of the primary cilia was significantly elongated, and at the time-point of 16 days after ischemia, the length of the primary cilia returned to the normal level (FIG. 4C).

Additionally, the presence and length of primary cilia in the distal tubular cells in the cortex and outer medulla were observed and the results are shown in FIG. 4D. According to the results, there was no significant change in the length of primary cilia in the distal tubular cells in the cortex and outer medulla at the time-point of 1 day after ischemia compared to that of the normal control, however, at the time-point of 8 days after ischemia, the length of the primary cilia in the distal tubular cells became longer than that of the normal controls. At the time-point of 16 days after ischemia, there was no change in the length of the primary cilia compared to that of the normal control, as shown in FIG. 4D.

Additionally, the presence and length of primary cilia in the collecting ducts in the cortex and the medulla were observed and the results are shown in FIG. 4E. According to the results, at the time-point of 24 hours after ischemia, the length of the primary cilia in the collecting ducts in the cortex and the medulla was significantly shortened compared to that of the normal control, whereas there was no significant change in the length of the primary cilia in the inner medullary cells compared to that of the normal kidney, as shown in FIG. 4E.

In particular, at the time-point of 8 days after ischemia, the length of the primary cilia in the collecting ducts of all nephron segments became longer than that of the normal control, and the length of the primary cilia in the collecting duct cells in the outer medulla was shown to be longest. At the time-point of 16 days after ischemia, the length of the primary cilia in the collecting ducts of all nephron segments returned to normal, as shown in FIG. 4E.

### Example 4: Detection of primary cilia fragments in urine after ischemia

Further to the confirmation of change in the primary cilia in renal tissues caused by the renal injury such as ischemia shown in Example 3, it was examined whether fragments of the primary cilia can be detached to be detected in the urine. In particular, antibodies specific to acetylated tubulin, the representative protein of the primary cilia, were used for the detection of the primary cilia.

As a result, as shown in FIG. 5, unlike in the sham-operated mice, acetylated tubulin was detected in the urine of mice which were induced with acute renal injury by ischemia/reperfusion. Specifically, acetylated tubulin was detected in the urine at the time-point of 4 hours after the ischemia/reperfusion injury, and it was increased at the time-point of 24 hours after the ischemia/reperfusion injury.

Furthermore, since the primary cilia have the characteristic to be supported by the basal bodies, the detection of ARL13B, the protein contained in the basal bodies, was examined as well. As a result, ARL13B was detected in the ischemia/reperfusion-operated mice, unlike in the sham-operated mice, at the time-point of 4 hours after the ischemia/reperfusion. Since ARL13b, due to its characteristic being a component of the basal bodies, is released later than the acetylated tubulin, it was thus detected later than the acetylated tubulin.

Additionally, β-actin, GAPDH, and CPI-17 (proteins present in cytoplasm, C-kinase-activated protein phosphatase-1 inhibitor) were not detected in the urine. This indicates that the primary cilia were detached before the cells were completely damaged, and thus the detection of the primary cilia, fragments thereof, or acetylated tubulin may serve as a marker for the sensitive detection of renal injuries.

Meanwhile, in order to confirm whether the amount of acetylated tubulin in the urine can serve as an index for determining the abnormalities of renal functions, the abnormalities in renal functions were examined in terms of plasma creatinine. The concentration of plasma creatinine increased as the renal injury became more severe (FIG. 5B). This indicates that the renal injuries became more severe as time passed. As shown in FIG. 5C, in the experiment, the concentration of plasma creatinine was positively correlated with the amount of excreted acetylated tubulin detected in the urine (r=0.94). This indicates that the amount of excreted acetylated tubulin may be used as a marker to determine the degree of a given renal disease.

### Example 5: Measurement of primary cilia length in regenerated cells

In order to confirm whether primary cilia are associated with cell proliferation, the present inventors examined the length of the primary cilia of the tubulin epithelial cells being regenerated. For the determination of the regenerating cells, BrdU was injected into mice 20 hours prior to kidney ablation. Since the BrdU is inserted into DNA during the S phase of a cell cycle, the insertion of BrdU will appear in the cells being proliferated. Accordingly, the length of the primary cilia of BrdU-positive cells, i.e., the length of the primary cilia in proliferative cells, was measured.

As a result, as shown in FIG. 6, the BrdU-positive cells were shown to have very short primary cilia or not have any primary cilia at all. This indicates that the investigation of the length of the primary cilia in renal tissues alone is not sufficient to determine the presence of a renal disease.

### Example 6: Effect of promoting normalization of primary cilia length by MnTMPyP treatment

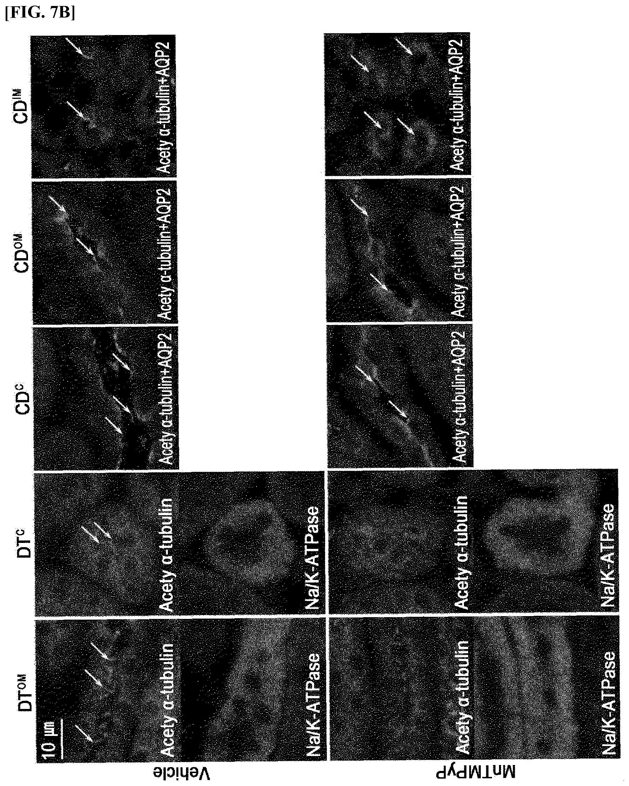

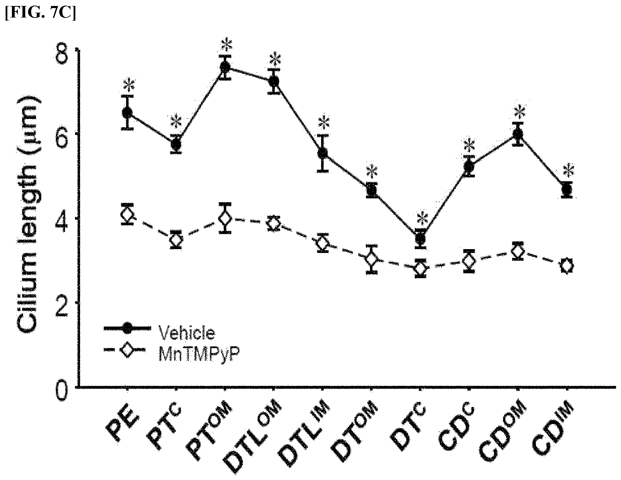

Regarding the change in the primary cilia after ischemia/reperfusion injury, in order to examine whether reactive oxygen species (ROS) are associated with the change in the length of primary cilia, the present inventors administered MnTMPyP, a peroxide remover, to mice daily for 6 days. In particular, the administration of MnTMPyP to the mice with the ischemia/reperfusion injury began at the time-point of 2 days after ischemia. Then, the level of superoxide and the length of primary cilia were examined and the results are shown in FIGS. 7 to 9. In the above figures, PE represents parietal cells, PTC represents a proximal tubule in the cortex, PTOM represents a proximal tubule in the outer medulla, DTLOM represents a descending thin limb in the outer medulla, DTLIM represents a descending thin limb in the inner medulla, DTOM represents a distal tubule in the outer medulla, DTC represents a distal tubule in the cortex, CDC represents a collecting duct in the cortex, CDOM represents a collecting duct in the outer medulla, and CDIM represents a collecting duct in the inner medulla.

As a result, the length of the primary cilia of the tubular epithelial cells in the outer medulla of the MnTMPyP-treated mice was shortened, compared to that of the mice in the control group (FIGS. 7A to 7C). The renal structure of the ischemia/reperfusion mice treated with MnTMPyP was improved compared to that of the normal group, and this indicates that the antioxidant treatment promotes the restoration of the length of the primary cilia altered by ischemia/reperfusion.

Additionally, the peroxide level in the kidneys of the MnTMPyP-treated mice was lower than that of the control group, and thus the anti-oxidative effect of MnTMPyP was confirmed (FIGS. 8A and 8B).

Then, considering that ROS activates ERK and the activated ERK is involved in the restoration of kidneys damaged by ischemia/reperfusion, the ERK activation and the change in the length of primary cilia were examined, and the results are shown in FIG. 9.

As a result, at the time-point of 8 days after ischemia, the levels of the phosphorylated ERK and the total ERK were increased, and these increases were significantly inhibited by MnTMPyP treatment (FIG. 9).

### Example 7: Detection of primary cilia fragments in culture after oxidative stress

In order to examine whether the shortening in the length of the primary cilia was due to deciliation, the present inventors confirmed acetylated α-tubulin as a marker for primary cilia fragments in a culture medium obtained from the control group or MDCK cells treated with hydrogen peroxide, and the results are shown in FIG. 10. Since hydrogen peroxide treatment shows a similar result to the method of renal injury caused by ischemia/reperfusion, it was thus used.

As a result, as shown in FIG. 10, acetylated α-tubulin was detected at the time-point of 4 hours after the hydrogen peroxide treatment and was increased with time. On the contrary, acetylated α-tubulin was not detected in the medium of the control group, and this indicates that deciliation occurred, in which the primary cilia were detached from the tubular cells in response to the oxidative stress.

### Example 8: Detection of primary cilia fragments in urine of patients with various renal diseases

In Examples 2, 5, and 7, it was confirmed that renal primary cilia were detected in an ischemia/reperfusion model-specific manner. Furthermore, the present inventors examined by Western blot analyses whether fragments of the primary cilia can be detected in the urine samples of patients with thin glomerular basement membrane (TGBM), minimal change disease (MCD), membranous glomerulonephritis (MGN), focal segmental glomerulosclerosis (FSGS), DM nephropathy, IgA nephropathy (IgAN), tubulointerstitial nephritis (TIN), Henoch-Schonlein Purpura nephritis (HSP nephritis), acute tubular injury, BK virus nephropathy, acute cellular rejection, chronic antibody-mediated rejection, chronic active antibody-mediated rejection, and chronic calcineurin inhibitor toxicity. The results are shown in FIGS. 11 to 16. In these figures, the patients' urine volume used in the Western blot analyses was 10 µL.

The results revealed that the primary cilia fragments were not detected in the urine of the normal control, whereas the primary cilia fragments were present in most urine samples of the patients with the renal diseases described above (FIGS. 11 to 16).

Additionally, the urine of a patient with kidney transplantation (allograft) was collected at preset time intervals after the transplantation, subjected to Western blot analysis, and it was confirmed that the primary cilia proteins were excreted into the urine during the early stage, i.e., at the time when the renal injury was severe. The results are shown in FIG. 17. In particular, the primary cilia protein was compared with the KIM-1 protein, and as a result, it was confirmed that the expression feature of the primary cilia protein was similar to that of KIM-1.

### Example 9: Detection of primary cilia fragments in urine from mouse model with type I diabetic nephritis and mouse model with acute hepatic injury

It was also examined whether the primary cilia fragments, i.e., proteins, can be detected in the urine of a mouse with type I DM nephropathy by Western blot analysis, and the results are shown in FIG. 18.

As a result, it was confirmed that the primary cilia fragments were detected in the urine of the mouse with type I DM nephropathy.

Additionally, the primary cilia fragments, i.e., proteins, were confirmed to be detected in the urine of a mouse induced with an acute hepatic injury by Western blot analysis, and the results are shown in FIG. 19.

As a result, it was confirmed that the primary cilia fragments were detected in the urine of the mouse induced with an acute hepatic injury.

That is, all the results shown in Examples indicate that various renal diseases can be diagnosed by specifically detecting primary cilia or fragments thereof in the urine. Additionally, because the presence of primary cilia can be detected even in a small amount of urine, it is suggested that the primary cilia isolated from urine, fragments thereof, or acetylated tubulin have high sensitivity and specificity in diagnosing various renal diseases.

From the foregoing, one of ordinary skill in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention.

## Claims

1. A method for diagnosing the occurrence or progress of a renal disease using a urine sample, comprising;
(i) contacting an isolated urine sample with (a) a binding agent specific for acetylated α-tubulin and, optionally, with (b) a binding agent specific for ARL13B, and
(ii) detecting the acetylated α-tubulin and, optionally, ARL13B; or the concentrations thereof from the isolated urine sample.

2. The method of claim 1, further comprising treating the isolated urine sample with a treating agent to decompose the primary cilia or the basal bodies of the primary cilia into proteins constituting the same, wherein the treating agent is selected from the group consisting of trypsin, chymotrypsin, plasmin, lysozyme, NP-40, SDS, Triton X-100, and SLS.

3. The method of claim 1, wherein the progress of a disease is diagnosed by investigating the quantitative change in the primary cilia and the primary cilia support, by treating the urine sample with both the agent for specifically binding to acetylated α-tubulin and the agent for specifically binding to ARL13B.

4. The method of claim 1, wherein the renal disease is selected from the group consisting of thin glomerular basement membrane (TGBM), minimal change disease (MCD), membranous glomerulonephritis (MGN), focal segmental glomerulosclerosis (FSGS), DM nephropathy, IgA nephropathy (IgAN), tubulointerstitial nephritis (TIN), Henoch-Schonlein Purpura nephritis (HSP nephritis), acute tubular injury, BK virus nephropathy, acute cellular rejection, chronic antibody-mediated rejection, chronic active antibody-mediated rejection, chronic calcineurin inhibitor (CIN) toxicity, acute kidney disease, injury in transplanted kidney, ischemic renal disease, glomerulonephritis, lupus nephritis, polycystic kidney disease, pyelonephritis, nephrolith, renal tuberculosis, renal tumor, chronic renal failure, end stage renal failure, sepsis, renal injury caused by hepatic injury, and a combination thereof.

5. The method of claim 1, comprising contacting an isolated urine sample with (a) a binding agent specific for acetylated α-tubulin and with (b) a binding agent specific for ARL13B.

6. The method of claim 1, further comprising detecting the presence of a renal disease from 1 µL to 50 µL of urine.

## Patentansprüche

1. Verfahren zum Diagnostizieren des Auftretens oder Fortschreitens einer Nierenerkrankung unter Verwendung einer Urinprobe, umfassend;
(i) Inkontaktbringen einer isolierten Urinprobe mit (a) einem Bindemittel, das für acetyliertes α-Tubulin spezifisch ist, und ggf. mit (b) einem Bindemittel, das für ARL13B spezifisch ist, und
(ii) Feststellen des acetylierten α-Tubulins und ggf. von ARL13B; oder deren Konzentrationen von der isolierten Urinprobe.

2. Verfahren nach Anspruch 1, ferner umfassend das Behandeln der isolierten Urinprobe mit einem Behandlungsmittel, um die primären Zilien oder die Basalkörper der primären Zilien in Proteine zu zerlegen, die selbige bilden, wobei das Behandlungsmittel ausgewählt ist aus der Gruppe bestehend aus Trypsin, Chymotrypsin, Plasmin, Lysozym, NP-40, SDS, Triton X-100 und SLS.

3. Verfahren von Anspruch 1, wobei das Fortschreiten einer Krankheit durch Untersuchen der quantitativen Veränderung in den primären Zilien und dem primären Zilien-Träger diagnostiziert wird, indem die Urinprobe sowohl mit dem Mittel zum spezifischen Binden des acetylierten α-Tubulins als auch mit dem Mittel zum spezifischen Binden mit ARL13B behandelt wird.

4. Verfahren nach Anspruch 1, wobei die Nierenerkrankung ausgewählt ist aus der Gruppe bestehend aus dünner glomerulärer Basalmembran (TGBM), Minimal-Change-Erkrankung (MCD), membranöser Glomerulonephritis (MGN), fokal segmentaler Glomerulosklerose (FSGS), DM-Nephropathie, IgA-Nephritis (IgAN), tubulointerstitieller Nephritis (TIN), Purpura Schönlein-Henoch-Nephritis (HSP-Nephritis), akuter tubulärer Verletzung, BK-Virus-Nephropathie, akuter Zellabstoßung, chronischer antikörpervermittelter Abstoßung, chronisch aktiver antikörpervermittelter Abstoßung, chronischer Calcineurin-Hemmer (CIN)-Toxizität, akuter Nierenerkrankung, Schädigung der transplantierten Niere, ischämischer Nierenerkrankung, Glomerulonephritis, Lupusnephritis, polyzystischer Nierenerkrankungen, Pyelonephritis, Nephrolith, renaler Tuberkulose, Nierentumor, chronischer Niereninsuffizienz, terminaler Niereninsuffizienz, Sepsis, durch Leberschädigung verursachte Nierenschäden, und einer Kombination davon.

5. Verfahren von Anspruch 1, umfassend das Inkontaktbringen einer isolierten Urinprobe mit (a) einem Bindemittel, das für acetyliertes α-Tubulin spezifisch ist, und mit (b) einem Bindemittel, das für ARL13B spezifisch ist.

6. Verfahren nach Anspruch 1, ferner umfassend das Feststellen des Vorliegens einer Nierenerkrankung in 1 µL bis 50 µL Urin.

## Revendications

1. Un procédé pour diagnostiquer l'apparition ou la progression d'une maladie rénale à l'aide d'un échantillon d'urine, comprenant:
(i) le fait de mettre en contact un échantillon d'urine isolé avec (a) un agent de liaison spécifique de l'α-tubuline acétylée et, éventuellement, avec (b) un agent de liaison spécifique de l'ARL13B, et
(ii) le fait de détecter l'α-tubuline acétylée et, éventuellement, de l'ARL13B; ou les concentrations de ces derniers dans l'échantillon d'urine isolé.

2. Le procédé selon la revendication 1, comprenant en outre le fait de traiter l'échantillon d'urine isolé avec un agent de traitement pour décomposer des cils primaires ou les corps basaux des cils primaires en des protéines constituant ceux-ci, l'agent de traitement étant choisi dans le groupe constitué par la trypsine, la chymotrypsine, la plasmine, le lysozyme, le NP-40, le SDS, le Triton X-100 et le SLS.

3. Le procédé selon la revendication 1, dans lequel la progression d'une maladie est diagnostiquée en étudiant le changement quantitatif des cils primaires et du support des cils primaires, en traitant l'échantillon d'urine avec à la fois l'agent pour la liaison spécifique à l'α-tubuline acétylée et l'agent pour la liaison spécifique à ARL13B.

4. Le procédé selon la revendication 1, dans lequel la maladie rénale est sélectionnée dans le groupe constitué par la membrane basale glomérulaire (MBG) mince, le syndrome néphrotique à lésions glomérulaires minimes (SNLGM), la glomérulonéphrite membraneuse (GNM), la glomérulosclérose segmentaire focale (FSGS), la néphropathie DM, la néphropathie à IgA (NIgA), la néphrite tubulo-interstitielle (NTI), la néphrite Purpura de Henoch-Schonlein (néphrite PHS), la lésion tubulaire aiguë, la néphropathie à virus BK, le rejet cellulaire aigu, le rejet chronique à médiation par anticorps, le rejet chronique à médiation par anticorps actifs, la toxicité chronique des inhibiteurs de la calcineurine (ICN), la maladie rénale aiguë, la lésion d'un rein transplanté, la maladie rénale ischémique, la glomérulonéphrite, la néphropathie lupique, la maladie polykystique des reins, la pyélonéphrite, la néphrolite, la tuberculose rénale, la tumeur rénale, l'insuffisance rénale chronique, l'insuffisance rénale terminale, la septicémie, la lésion rénale causée par une lésion hépatique, et une combinaison de ces maladies.

5. Le procédé selon la revendication 1, comprenant le fait de mettre en contact un échantillon d'urine isolé avec (a) un agent de liaison spécifique de l'α-tubuline acétylée et avec (b) un agent de liaison spécifique de l'ARL13B.

6. Le procédé selon la revendication 1, comprenant en outre le fait de détecter la présence d'une maladie rénale dans 1 µL à 50 µL d'urine.
